# NEW EUROPEAN PATENT SPECIFICATION

(11) **EP 2 809 182 B2**
(45) Date of publication and mention of the opposition decision: **19.01.2022**
(45) Mention of the grant of the patent: 31.01.2018
(21) Application number: 13743051.8
(22) Date of filing: 31.01.2013
(51) Int. Cl.: A61M 15/06, A24F 40/485, A24F 40/10, A61M 11/04, A61M 21/00, A61M 16/00

(54) **ELECTRONIC SMOKING ARTICLE**
ELEKTRONISCHER RAUCHARTIKEL
ARTICLE À FUMER ÉLECTRONIQUE

(30) Priority: 31.01.2012 US 201261593004 P
(43) Date of publication of application: 10.12.2014
(73) Proprietor: Altria Client Services LLC, Richmond, Virginia 23230 (US)
(72) Inventor: LI, San, Midlothian, Virginia 23114 (US); KARLES, Georgios, Richmond, Virginia 23233 (US); MISHRA, Munmaya K., Manakin Sabot, Virginia 23103 (US); LI, Weiling, Moseley, Virginia 23120 (US); SMITH, Barry S., Hopewell, Virginia 23860 (US); ROSTAMI, Ali A., Glen Allen, Virginia 23059 (US); TUCKER, Christopher S., Midlothian, Virginia 23114 (US); JORDAN, Geoffrey Brandon, Midlothian, Virginia 23112 (US)
(74) Representative: Docherty, Andrew John
(86) International application number: PCT/US2013/024224
(87) International publication number: WO 2013/116568

(56) References cited:
- WO-A1-2009/135729
- WO-A1-2010/107613
- CN-U- 201 860 753
- CN-U- 201 860 753
- US-A1- 2006 196 518
- US-A1- 2006 196 518
- US-A1- 2007 267 032
- US-A1- 2007 267 032
- US-A1- 2008 047 571
- US-A1- 2008 047 571
- US-A1- 2008 230 052
- US-A1- 2008 230 052
- US-A1- 2010 242 975
- US-A1- 2010 242 975
- US-A1- 2011 094 523
- US-A1- 2011 094 523

## Description

This application concerns an electronic smoking article. The prior art (see CN 201 860 753 U or US 2011/094523 A1) describes such a device, mainly comprising an outer tube, an aerosol generator provided with a liquid supply.

### Summary of Selected Features

An electronic cigarette or cigar (collectively "electronic smoking article") is provided which includes a heater element which vaporizes liquid material to produce an aerosol or "vapor". In an embodiment, the heater element comprises a resistive heater coil, with a wick extending therethrough. Aerosol generated by the heater coil and wick assembly is drawn down a central channel toward the mouth end portion of the smoking article.

The electronic article according to the invention includes a mechanical aerosol converter (MAC) insert having a face and one or more outlets. The face and outlets of the MAC insert are mutually arranged and the face is aligned with the central channel so as to cause the aerosol to strike the face prior to it being drawn out from the article. As a result, the aerosol particle size distribution is shifted to a distribution comprising a range of smaller particles, and both the vapor phase components of the aerosol and the temperature of the aerosol are reduced. These effects and possibly others are believed to contribute to sensory attributes of reduced throat irritation, even at higher levels of nicotine content in the liquid formulation, and to improved mouth feel over aerosols of electronic smoking articles lacking a MAC insert as taught herein.

### Brief Description of the Drawings

Figures 1A and 1B are cross sectional side views of an electronic smoking article according to a first embodiment;
Figure 2 is a perspective view of a mechanical aerosol converter (MAC) insert of the electronic smoking article shown in Figure 1A, the perspective being viewed in the general direction of arrow A in Figure 1A;
Figures 3A and 3B are perspective views of MAC inserts of Figures 1A and 1B, the perspective being viewed in the general direction of arrow B in Figures 1A and 1B;
Figure 4 is a side view of the MAC insert of Figures 1A, 2 and 3A, with interior features of the face shown in dashed lines;
Figure 5A is a perspective view of an another embodiment of a MAC insert including a flange for use in the electronic smoking article shown in Figure 1A, the perspective being viewed in the general direction of arrow B in Figure 1A;
Figure 5B is a perspective view of an another embodiment of a MAC insert excluding a flange for use in the electronic smoking article shown in Figure 1B, the perspective being viewed in the general direction of arrow B in Figure 1B;
Figure 6 is an abbreviated sectional side view of an electronic smoking article in accordance with another embodiment including a flow-centralizer disc interposed between a first MAC insert, shown in Figure 5A, and a second MAC insert, shown in Figure 5B;
Figure 7 is a perspective view of the flow-centralizing disc of Figure 6;
Figure 8 is a stylized representation of a possible effect of aerosol striking a MAC insert prior to exiting an electronic smoking;
Figure 9 is an abbreviated sectional side view of an electronic smoking article having a mouth end insert constructed in accordance with another embodiment, and including a flow-centralizer disc interposed between an upstream MAC insert, as shown in Figure 5B, and a downstream, multi-ported mouthpiece insert;
Figures 10A and 10B are perspective views of a multi-ported mouth end insert for use in the electronic smoking article of Figure 9;
Figure 11 is an abbreviated sectional side view of an electronic smoking article having a MAC insert constructed in accordance with another embodiment; wherein the MAC insert is in the form of a disc.
Figure 12 is a planar view of the MAC insert as shown in Figure 11;
Figure 13 is sectional side view of a detail of a gasket having a convergent outlet for inclusion in any of the foregoing embodiments;
Figure 14 is a cross sectional view of a MAC insert including a plurality of partitions therein; and
Figure 15 is an abbreviated cross sectional side view of an electronic smoking article including another embodiment of a MAC insert.

### Electronic Smoking Article Layout

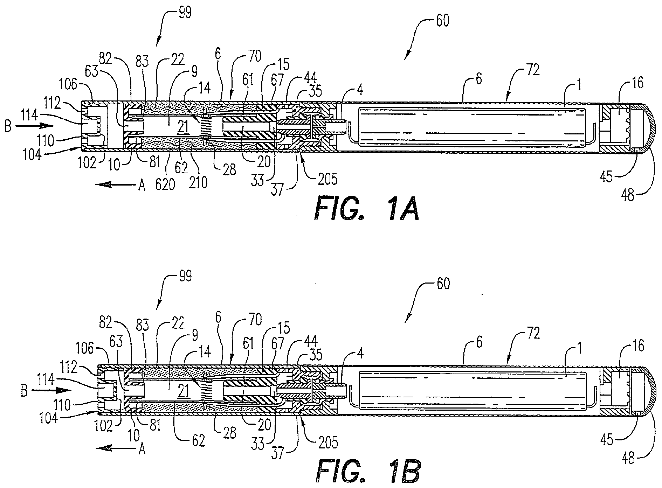

Referring to Figures 1A and 1B, an electronic smoking article (cigarette) 60 comprises a replaceable cartridge (or first section) 70 and a reusable fixture (or second section) 72, which in a preferred embodiment, are coupled together at a threaded connection 205 or by other convenience such as a snug-fit, detent, snap-fit, clamp and/or clasp. Generally, the second section 72 includes a puff sensor 16 responsive to air drawn into the second section 72 via an air inlet port 45 adjacent the free end or tip of the electronic smoking article 60, a battery 1 and control circuitry. The disposable first section 70 includes a liquid supply region 22 of liquid and a heater 14 that aerosolizes liquid that is drawn from the liquid supply region 22 through a wick 28. Upon completing the threaded connection 205, the battery 1 is connectable with the electrical heater 14 of the first section 70 upon actuation of the puff sensor. Air is drawn primarily into the first section 70 through one or more air inlets 44 located in the outer tube (casing) 6.

In a preferred embodiment, once the liquid of the cartridge is spent, only the first section 70 is replaced. An alternate arrangement includes a layout where the entire electronic smoking article 60 is disposed once the liquid supply is depleted. In such case the battery type and other features might be engineered for simplicity and cost-effectiveness, but generally embodies the same concepts as in the preferred embodiment in which the second section is reused and/or recharged.

In a preferred embodiment, the electronic smoking article 60 is about the same size as a conventional cigarette. In some embodiments, the electronic smoking article 60 can be about 80 mm to about 110 mm long, preferably about 80 mm to about 100 mm long and about 7 mm to about 8 mm in diameter. For example, in a preferred embodiment, the electronic smoking article is about 84 mm long and has a diameter of about 7.8 mm.

Preferably, at least one adhesive-backed label is applied to the outer tube 6, preferably about the first section 70. The label preferably completely circumscribes the electronic smoking article 60 and can be colored and/or textured to provide the look and/or feel of a traditional cigarette. The label can include holes therein which are sized and positioned so as to prevent blocking of the air inlets 44 in the outer tube 6 (or casing).

The outer tube 6 and/or the inner tube 62 may be formed of any suitable material or combination of materials. Examples of suitable materials include metals, alloys, plastics or composite materials containing one or more of those materials, or thermoplastics that are suitable for food or pharmaceutical applications, for example polypropylene, polyetheretherketone (PEEK), ceramic, and polyethylene. Preferably, the material is light and non-brittle. The inner tube 62 can also include a substantially water impermeable coating.

Referring now to Figures 1A, 1B, 6 and 9, the first section 70 includes the outer tube (or casing) 6 extending in a longitudinal direction and the inner tube (or chimney) 62 coaxially positioned within the outer tube 6. Preferably, a nose portion 61 of an upstream gasket (or seal) 15 is fitted into an upstream end portion 65 of the inner tube 62, while at the same time, an outer perimeter 67 of the upstream gasket 15 provides a liquid-tight seal with an interior surface of the outer tube 6. The upstream gasket 15 also includes a central, longitudinal air passage 20, which opens into an interior of the inner tube 62 that defines a central channel 21.

Referring to Figure 1, a transverse channel 33 can be provided across a backside potion of the upstream gasket 15, which intersects and communicates with the central channel 20 of the gasket 15. This channel 33 assures communication between the central channel 20 and a space 35 defined within a cathode connector piece 37. Optionally, the piece 37 includes a threaded section for effecting the threaded connection 205. The cathode connector piece 37 includes opposing notches about its perimeter, which, upon insertion of the cathode connector piece 37 into the outer tube 6, are aligned with the location of each of two RTD-controlling, air inlet ports 44 and 44' in the outer tube 6. Preferably, the air inlet ports 44 and 44' are precision drilled so as to provide the smoking article a predetermined, desired resistance to draw (RTD) ranging from about 60 mm H₂O to about 150 mm H₂O, more preferably about 90 mm H₂O to about 110 mm H₂O, most preferably about 100 mm H₂O to about 130 mm H₂O.

The space defined between the upstream gasket 15, a downstream gasket 10 and the outer tube 6 and the inner tube 62 establish the confines of the liquid supply region 22. The liquid supply region 22 comprises a liquid material and optionally a liquid storage medium 210 operable to store the liquid material therein. The liquid storage medium 210 may comprise a winding of cotton gauze or other fibrous material about the inner tube 62.

Optionally, the liquid supply region 22 is contained in an outer annulus 620 between inner tube 62 and outer tube 6 and between the gaskets 10 and 15. Thus, the liquid supply region 22 at least partially surrounds the central air passage 21. The heater 14 preferably extends transversely across the central channel 21 between opposing portions of the liquid supply region 22, although the teachings herein are applicable to arrangements wherein the heater 14 is oriented in the longitudinal direction instead of transversely.

Preferably, the liquid storage medium 210 is a fibrous material comprising cotton, polyethylene, polyester, rayon and combinations thereof. Preferably, the fibers have a diameter ranging in size from about 6 microns to about 15 microns (e.g., about 8 microns to about 12 microns or about 9 microns to about 11 microns). The liquid storage medium 210 can be a sintered, porous or foamed material. Also preferably, the fibers are sized to be irrespirable and can have a cross-section which has a y shape, cross shape, clover shape or any other suitable shape. In the alternative, the liquid supply region 22 may comprise a filled tank lacking a fibrous storage medium 21 and containing only liquid material.

Also preferably, the liquid material has a boiling point suitable for use in the electronic smoking article 60. If the boiling point is too high, the heater 14 will not be able to vaporize liquid in the wick 28. However, if the boiling point is too low, the liquid may vaporize even when the heater 14 is not being activated.

Preferably, the liquid material includes a tobacco-containing material including volatile tobacco flavor compounds which are released from the liquid upon heating. The liquid may also be a tobacco flavor containing material or a nicotine-containing material. Alternatively, or in addition, the liquid may include a non-tobacco material. For example, the liquid may include water, solvents, ethanol, plant extracts and natural or artificial flavors. Preferably, the liquid further includes an aerosol former. Examples of suitable aerosol formers are glycerine and propylene glycol.

In use, liquid material is transferred from the liquid supply region 22 and/or liquid storage medium 210 by capillary action at each end portion of the wick 28. In an embodiment, the heater 14 can at least partially surround a central portion of the wick 28 such that when the heater 14 is activated, the liquid in that portion of the wick 28 is vaporized by the heater 14 to form an aerosol. In other embodiments, the heater 14 can be disposed adjacent a portion of the wick without being wound about the wick. The wick 28 preferably comprises filaments having a capacity to draw a liquid, more preferably a bundle of glass (or ceramic) filaments and most preferably a bundle comprising a group of windings of glass filaments, preferably three of such windings, all which arrangements are capable of drawing liquid via capillary action via spacings between the filaments. Preferably, the wick 28 is flexible and includes three strands, each strand including a plurality of filaments. Moreover, it is noted that the end portions of the wick 28 are preferably flexible and foldable into the confines of the liquid supply region 22.

Preferably, a nose portion 81 of a downstream gasket 10 is fitted into a downstream end portion 83 of the inner tube 62. An outer perimeter 82 of the gasket 10 provides a substantially liquid-tight seal with an interior surface of the outer tube 6. Aerosol generated by the heater 14 travels from the central channel 21 and through a central channel 63 in the downstream gasket 10 and into a remaining downstream end portion of a mouth piece portion 99 of the smoking article 60. From the central channel 63 of the gasket 10, the aerosol, in substantial part, is drawn into contact with a face 102 of a mechanical aerosol converter (MAC) insert 104.

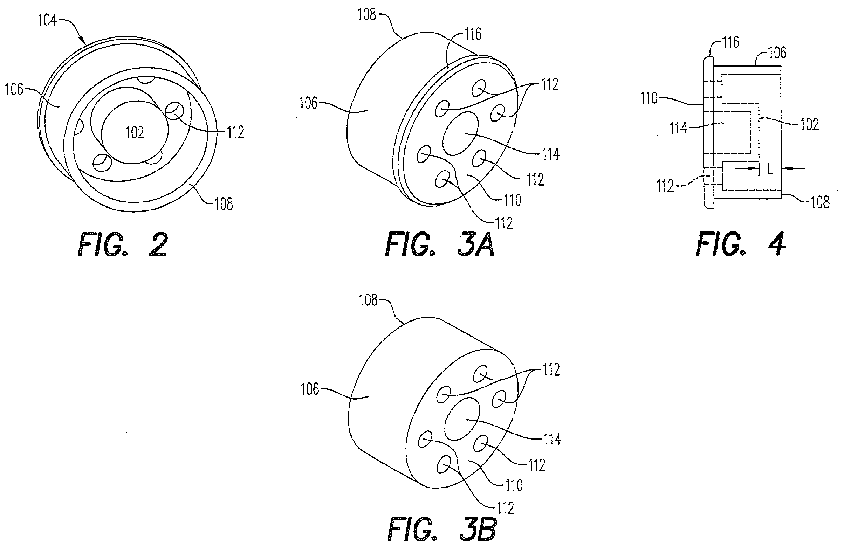

Referring now to Figure 2, in one embodiment, the MAC insert 104 comprises a cylindrical body portion 106 having an outer diameter that can be sized to provide a sliding fit with the interior surfaces of the outer tube 6 of the smoking article 60. As shown in Figure 1B, an upstream end portion 108 of the cylindrical body portion 106 of the MAC insert 104 is preferably brought into contact with adjacent portions of the gasket 10 so that the spatial relationship and orientation between the MAC insert 104 and the gasket 10 are essentially the same from one article 60 to the next. Thus, the body portion 106 is provided with a length such that a face portion 110 of the MAC insert 104 is flush with or at some other desired relation with the downstream end of the outer tube 6. The MAC insert 104 can be formed of any suitable substantially air impermeable material, such as plastic or metal foil.

The transverse, annular face portion 110 of the MAC insert 104 is provided with a plurality of orifices 112, which in an embodiment comprise six circular orifices 112, each orifice having a diameter ranging from about 0.015 inch to about 0.090 inch (e.g., about 0.020 inch to about 0.040 inch or about 0.028 inch to about 0.038 inch). After contacting the face 102 of the MAC insert 104, aerosol is then drawn through the orifices 112 in the MAC insert 104, which are disposed radially about the face 102.

Although the orifices 112 are shown as extending in a longitudinal direction, all or some of them may be directed divergently so as to impart a radially outward component to the velocity to the aerosol streams as they are drawn through the MAC insert 104. The number, size and shape of the orifices can be varied in the practice of the teachings herein.

Referring now to Figures 2 and 4, the face 102 of the MAC insert 104 is disposed within the confines of the cylindrical body portion 106 by a predetermined spacing "L" from the edge portion 108 of the cylindrical body portion 106 such that upon placement of the MAC insert 104 against the gasket 10, the face 102 is positioned a predetermined distance apart from the discharge orifice 63 of the gasket 10. In a preferred embodiment, that distance is in the range of about 1 mm to about 3 mm, more preferably in the range of about 1.5 mm to about 2.5 mm, but could be established at other values through analytic testing and/or modeling to find which distance is preferred for any particular design of the electronic smoking article 60.

Referring to Figures 3A, 3B, and 4, the MAC insert 104 can include a central recess 114 to facilitate molding operations during the manufacture of the MAC insert 104. It is envisioned that the MAC insert 104 could be formed without the recess 114 such that an external face portion 110 of the insert is generally circular and planar instead of being annular as shown in Figures 3A and 3B. In addition, the MAC insert 104 may be provided with a flange 116 (as shown in Figure 3A) which could act as a stop against the downstream edge of the outer tube 6 so as to register placement within the smoking article 60 with the flange 116 instead of abutment against the insert 10 as shown in Figure 1A. Such arrangement is advantageous when it is desired to locate the MAC insert 104 in spaced apart relation from the gasket 10. It is also envisioned that the MAC insert 104 and the gasket 10 can be integrally formed so as to simplify manufacture and assembly of the electronic smoking article 60.

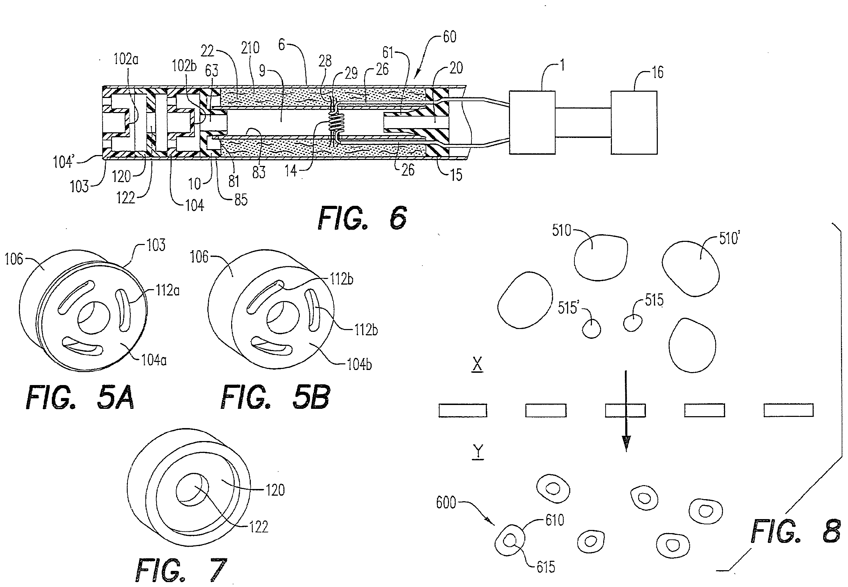

Referring now to Figures land 8, as aerosol is drawn through the central channel 63 of the gasket 10, it is directed against the face 102 of the MAC insert 104, whereupon heat is exchanged such that the temperature of the aerosol is reduced. In some cases, the temperature can be reduced by 30°C or more. When the aerosol strikes the face 102, larger aerosol particles, as represented in region X of Figure 8, tend to break up to form several smaller particles, as represented in region Y.

In particular and in reference to region X of Figure 8, it is believed for that upon exiting the central channel 63 of the gasket 10, the aerosol comprises large particles of propylene glycol and water 510 and relatively large, separate particles of nicotine droplets 515. The relatively large, nicotine droplets 515 comprise a vapor phase component of the aerosol and are known to contribute undesirable sensory responses such as throat irritation and/or harshness when inhaled.

Not wishing to be bound by theory, upon striking the face 102 of the MAC insert 104, it is believed that the aerosol undergoes a transformation such as shown in region Y of Figure 8, whereby larger particles 510 and 515 of the aerosol as previously described become smaller particulate phase constituents 600 comprising smaller, particulate phase nicotine 615 surrounded by propylene glycol 610.

As a result of drawing the aerosol through the MAC insert 104, it has been found that the aerosol of the electronic smoking article 60 tends to be lower in temperature, to have a particle size distribution that has shifted toward a range of smaller particle sizes and that the vapor phase components as previously described are reduced. It is also believed that the MAC insert 104 increases production of core-shell particles comprising a nicotine particle surrounded by a shell of propylene glycol. These attributes and others are believed to contribute to a finding that the aerosol has acceptable sensory attributes including less throat irritation and improved sensory experience, even at higher nicotine levels in the liquid formulation. The reduced aerosol temperature is believed to improve mouth feel.

As noted, use of the MAC insert 104 tends to avoid perceived sensory deficits when elevated levels of nicotine are added to the liquid formulation of an electronic smoking article. For example, an electronic smoking article of the prior art tends to produce perceived sensations of harshness and/or irritation if the liquid formulation is modified to include nicotine at levels greater than 2% by weight. It has been found that with inclusion of a MAC insert in accordance with the teachings herein, nicotine levels in the liquid formulation can be increased up to 6% nicotine with acceptable sensory attributes. Such provides opportunity to produce an electronic smoking article 60 having high impact with little to no irritation. With inclusion of the MAC insert 104, an electronic smoking article can be constructed which provides 0.18 mg of nicotine per puff on a 10 puff basis, which provides comparable levels of nicotine when compared to traditional cigarettes, with acceptable sensory attributes.

With regard to particle size distribution, it has been found that particles size distribution of an aerosol from a smoking article lacking a MAC insert as taught herein has a bell-shaped distribution of sizes in the range of about 0.1 nanometer (nm) to about 0.7 nm. In contrast, the particles size distribution of an aerosol from an electronic smoking article including the MAC insert as described herein has a bell-shaped distribution of sizes in the range of about 0.1 nm to about 0.6 nm, a significant decrease in particle size distribution.

It has also been found that some electronic smoking articles not including a MAC insert tend to produce aerosols having temperatures at or above 110° Celsius. In contrast, an electronic smoking article 60 as described in Figures 1A and 1B, which include the MAC insert 104, produce an aerosol having a temperature, when measured at the exit of the mouth end of the electronic smoking article 60, of about 60° C to about 100° C.

Referring now to Figure 5A, according to another embodiment, the MAC insert 104a, 104b includes three exit orifices 112a, 112b that are elongated, generally kidney shaped and extend circumferentially about the perimeter of the face 102a, 102b of the MAC insert 104a, 104b. Being larger, the orifices 112a, 112b are less subject to risk of accumulation of material and blockage. In a preferred embodiment, each orifice 112a, 112b has a length of about 2 mm to about 5 mm, preferably about 3 mm to about 4 mm, and a width of about 0.5 mm to about 1 mm in width. The face 102a, 102b can have a diameter of about 3 mm to about 5 mm. The size, circumferential extent and the number of the orifices 112a, 112b may be varied in the practice of these teachings with respect to any particular article.

Referring now to Figure 6, another embodiment of an electronic smoking article 60 comprises many of the same components as described with reference to Figures 1A and 1B, but with the battery section 1 and the puff sensors/control circuitry 16 shown in block diagram. In this embodiment, a first MAC insert 104b is disposed immediately downstream of the gasket 10 as previously described with reference to Figure 1. A disk 120 having a central orifice 122 is positioned downstream of the first MAC insert 104b and immediately upstream of a second MAC insert 104a.

The disc 120 with its central orifice 122 operates to centralize the discharge of the first MAC insert 104b before it is drawn through the second MAC insert 104a. In essence the flow-centralizing disc imparts additional compaction of the aerosol while also directing the flow against the face 102a of the second MAC insert 104a. With this arrangement, additional mechanical aerosol converting events are imparted on the aerosol so as to further enhance the beneficial aspects of reduction of aerosol particle size upon the aerosol generated by the electronic smoking article 60.

It is envisioned that the first MAC insert 104b may have orifices of the shape such as shown in Figure 2, whereas the second MAC insert 104a might have the elongated orifices shown in Figures 5A or 5B or vise-versa. Alternatively, both the first MAC insert 104b and the second MAC insert 104a can include the round orifices shown in Figures 2 and 3 or both can include the elongated orifices shown in Figures 5A and 5B. Moreover, the second MAC insert 104a can include a flange 103 as shown in Figure 5A, while the first MAC insert 104b may exclude a flange.

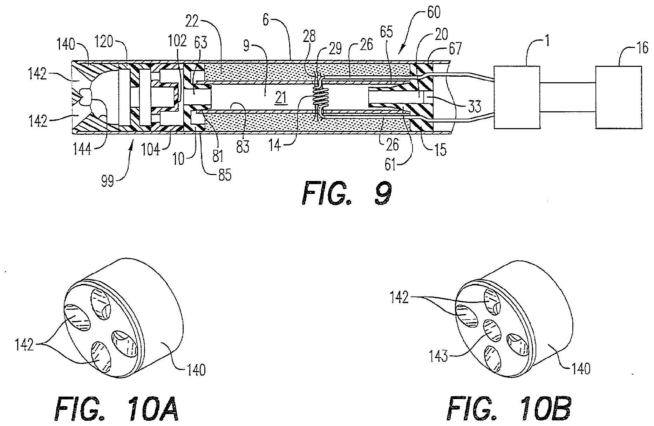

Referring now to Figure 9, another embodiment provides a smoking article as previously described including a downstream gasket 10 followed by (in the sense of direction of aerosol flow), a MAC insert 104, 104a, 104b, as shown in Figures 2, 5A or 5B, which is in turn is followed by a disk 120, as previously described, which in turn is followed by a multiport mouth end insert 140 having a plurality of divergent passages 142. Referring now also to Figure 10A, the multi-port mouth end insert 140 is constructed in accordance with the teachings of U.S. Application Serial Number 13/741,217 to Tucker et al. filed January 14, 2013 and titled "Electronic Cigarette", which has been published on August 1, 2013 with No. US 2013/0192615 A1, after the filing date of this application.

In a preferred embodiment, the multi-port mouth end insert 140 includes at least two diverging outlet passages 142 (e.g., preferably 2 to 10 outlet passages 142, more preferably 4 outlet passages or 3, 4, 5, 6, or more passages 142). Preferably, the outlet passages 142 are located off-axis and are angled outwardly in relation to the central channel 21 of the inner tube 62 (i.e., divergently). Also preferably, the outlets 142 are uni formly distributed about a perimeter of the insert 140 so as to substantially uniformly distribute aerosol in a smoker's mouth during use and create a greater perception of fullness in the mouth. Thus, as the aerosol passes into a smoker's mouth, the aerosol enters the mouth and moves in divergent directions so as to provide a full mouth feel. In contrast, electronic smoking articles having a single, on-axis orifice tend to direct its aerosol as single jet of greater velocity toward a more limited location within a smoker's mouth.

In addition, the multi-ported mouthpiece insert 140 and its diverging outlet passages 142 are arranged and include interior surfaces 144 such that droplets of un-aerosolized liquid material, if any, that may be entrained in the aerosol strike the interior surfaces 144 of the mouth end insert 140 and/or strike portions of walls of the diverging outlet passages 142. As a result such droplets are substantially removed or broken apart so as to enhance o\the aerosol.

Optionally, the diverging outlet passages 142 are angled at about 5° to about 60° with respect to the longitudinal axis of the outer tube 6 so as to more completely distribute aerosol throughout a mouth of a smoker during use and to remove droplets. In a preferred embodiment, there are four diverging outlet passages 142, each at an angle of about 40° to about 50° with respect to the longitudinal axis of the outer tube 6, more preferably about 40° to about 45° and most preferably about 42°.

Preferably, each of the diverging outlet passages 142 has a diameter ranging from about 0.015 inch to about 0.090 inch (e.g., about 0.020 inch to about 0.040 inch or about 0.028 inch to about 0.038 inch). The size of the diverging outlet passages 24 and the number of diverging outlet passages 24 can be selected to adjust the resistance to draw (RTD) of the electronic smoking article 60, if desired. Moreover, the mouth end insert 8 can be formed of a polymer selected from the group consisting of low density polyethylene, high density polyethylene, polypropylene, polyvinylchloride, polyetheretherketone (PEEK) and combinations thereof. The mouth end insert 8 may also be colored if desired.

In one embodiment, shown in Figure 10B, the mouth end insert 140 can include an on-axis central outlet passage 143, together with the diverging outlet passages 142.

As shown in Figure 9, an interior surface 144 of the mouth end insert 140 can comprise a generally domed surface 144. Alternatively, the interior surface 144 of the mouth end insert 140 can be generally cylindrical or frustoconical, with a planar end surface. Preferably, the interior surface 144 is substantially uniform over the surface thereof. Moreover, the interior surface 144 can be symmetrical about the longitudinal axis of the mouth end insert 140. However, in other embodiments, the interior surface 140 can be irregular and/or have other shapes.

In a preferred embodiment, the interior surfaces 144 of the insert 140 may include a void disposed at the convergence of the diverging outlet passages 144.

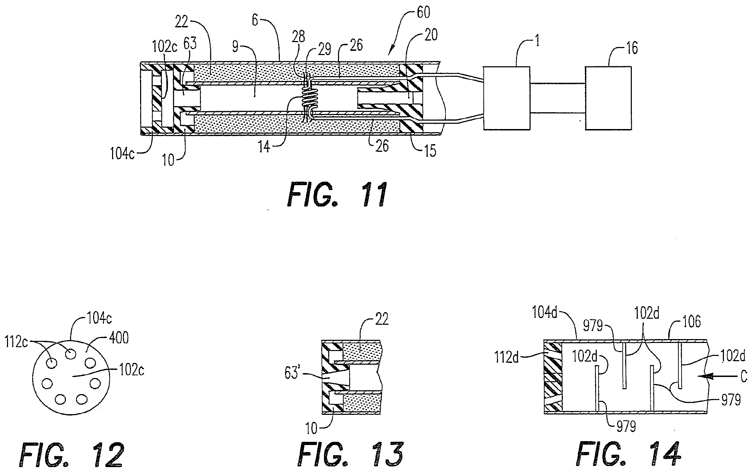

Referring now to Figure 11, in another embodiment, the electronic smoking article 60 includes a gasket 10 having a central channel 63 as previously described with the other embodiments, together with a MAC insert 104c, which functions in a manner similar to that described with respect to the MAC insert 104 of the first embodiment with reference to Figures 1A and 1B. However, the MAC insert 104c of embodiment in Figure 11 is in the form of a single disc having openings 112c radially spaced outwardly of a central face 102c. In this embodiment, the openings 112c and the face 102c are coplanar, whereas in the first embodiment shown in Figures 1A, 1B, 2 and 3 , the face 102 is spaced upstream from the openings 112. Each opening 112c may have a diameter ranging from about 0.015 inch to about 0.090 inch (e.g., about 0.020 inch to about 0.040 inch or about 0.028 inch to about 0.038 inch).

Referring now to Figure 13, in that higher velocities may enhance the benefits of impaction, expedients may be implemented to increase the speed of the aerosol as it approaches the face 102 of the MAC insert 104. For example, the gasket 10 can include a constricted central channel 63' to produce greater velocities in the aerosol as it is drawn through the central channel 63', and acts as an aerosol stream forming element.

Referring now to Figure 14, in another embodiment, the MAC insert 104d comprises a cylindrical body 106 and a series of partitions 979 extending partially across the an interior of the cylindrical body 106 in alternating relationship. The partitions 979 are longitudinally spaced from one another and present multiple faces 102d, which aerosol may strike as aerosol passes through the MAC insert 104d. In this embodiment, the outlet orifices 112d are provided downstream of the partitions 979.

Optionally, the orifices 112, 112a, 112b, 112c, 112d, 112e in the embodiments of Figures 2, 3, 5A, 5B, 12, 14 and 15 may be sized to provide an RTD controlling aspect.

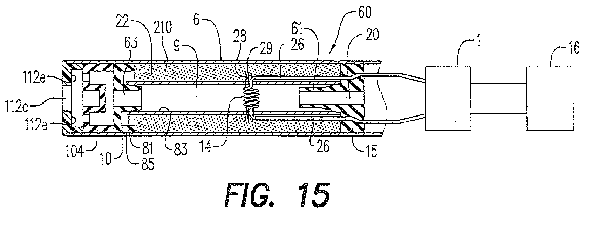

Optionally, as shown in Figure 15, it is contemplated that the relative, radial positions of the face 102e and the exit orifices 112e can be reversed such that an aerosol may be directed instead to an outer peripheral region of the MAC insert 104e and drawn through a central orifice 112e.

Optionally, the face 102, 102a, 102b, 102c, 102d, 102e of the MAC insert 104, 104a, 104b, 104c, 104d, 104e may be porous such that it has the capacity to collect particles through adsorption and/or absorption and in lieu of or in addition, may be concave in the upstream direction or in the downstream direction to adjust its characteristics as a mechanical aerosol converter.

Although the above teachings are with reference to a particular layout of an electronic smoking article 60, the teachings are equally applicable to any electronic smoking article whatever the configuration.

When the word "about" is used in this specification in connection with a numerical value, it is intended that the associated numerical value include a tolerance of ± 10% around the stated numerical value. Moreover, when reference is made to percentages in this specification, it is intended that those percentages are based on weight, i.e., weight percentages.

Moreover, when the words "generally" and "substantially" are used in connection with geometric shapes, it is intended that precision of the geometric shape is not required but that latitude for the shape is within the scope of the disclosure. When used with geometric terms, the words "generally" and "substantially" are intended to encompass not only features which meet the strict definitions but also features which fairly approximate the strict definitions.

It will now be apparent that a new, improved, and nonobvious electronic smoking article has been described in this specification with sufficient particularity as to be understood by one of ordinary skill in the art. Moreover, it will be apparent to those skilled in the art that numerous modifications, variations, substitutions, and equivalents exist for features of the electronic smoking article which fall within the scope of the invention as defined by the appended claims.

## Claims

1. An electronic smoking article (60) comprising:
an outer tube (6) extending in a longitudinal direction;
an aerosol generator within the outer tube (6) which in use produces a condensation aerosol within the outer tube (6);
a liquid supply (22) within the outer tube (6) for delivering liquid material to the aerosol generator; and
an aerosol stream forming element within the outer tube (6) operable to produce an aerosol stream, wherein the aerosol stream forming element is a gasket (10) arranged such that in use the aerosol is drawn through a central orifice (63) of the gasket (10);
**characterized in that** the electronic smoking article (60) further comprises:
a mouth end insert in the form of a mechanical aerosol converter insert (104) disposed downstream of said gasket (10), said mechanical aerosol converter insert (104) arranged to impart impaction upon said aerosol stream as said aerosol is being drawn from said electronic smoking article (60), the mechanical aerosol converter insert (104) including three elongated, generally kidney shaped, exit orifices (112) extending circumferentially about a perimeter of an exposed end surface (110) of the mechanical aerosol converter insert (104) and extending circumferentially about the perimeter of a face, of the mechanical aerosol converter insert (104), said comprising face (102) disposed upstream of said exposed end surface (110), said face (102) being spaced from said central orifice (63), said face (102) and said central orifice (63) being arranged such that when in use at least some of said aerosol stream drawn through said central orifice (63) strikes said face (102), and a characteristic of said aerosol is altered.

2. The electronic smoking article of claim 1, wherein the three elongated exit orifices (112) extending circumferentially about the perimeter of the exposed end surface (110) are disposed radially outward of said face (102).

3. The electronic smoking article of claim 2, wherein said face (102) is disposed within a cylindrical body (106), said face (102) is spaced from an upstream edge portion (108) of said cylindrical body (106), said edge portion (108) abutting said gasket (110), wherein spacing between said face (102) and said central orifice (63) is consistently established.

4. The electronic smoking article of claim 1, wherein the characteristic of said aerosol is at least one of aerosol temperature and particle size distribution.

5. The electronic smoking article of claim 1, further comprising:
an inner tube (62) within the outer tube (6), wherein the aerosol generator comprises a heater (14) located in the inner tube (62); and
a wick (28) in communication with the liquid supply (22) and surrounded by the heater (14) such that in use the wick (28) delivers liquid material to the heater (14) and the heater (14) heats the liquid material to a temperature sufficient to vaporize the liquid material and form an aerosol in the inner tube (62), wherein the liquid supply (22) is contained in an outer annulus (620) between the outer tube (6) and the inner tube (62).

6. The electronic smoking article of claim 1, wherein the face (102) is porous and particles of a predetermined size can be retained on the face (102).

7. The electronic smoking article of claim 1, wherein the face (102) is concave.

8. The electronic smoking article of claim 5, wherein the electronic smoking article (60) comprises a first section (70) attachable to a second section (72) and wherein the wick (28), the heater (14) and the liquid supply (22) are contained in the first section (70) and a power supply (1) is contained in the second section (72).

9. The electronic smoking article of claim 1, wherein the liquid supply (22) has a nicotine content of greater than 2% by weight based on the weight of the liquid supply (22).

10. The electronic smoking article of claim 1, wherein each of the three elongated exit orifices (112) has a length ranging from about 2 mm to about 5 mm and a width ranging from about 0.5 mm to about 1 mm.

11. The electronic smoking article of claim 1, wherein the mechanical aerosol converter insert (104) is formed of plastic or metal foil.

## Patentansprüche

1. Elektronischer Rauchartikel (60), umfassend:
ein äußeres Rohr (6), das sich in einer Längsrichtung erstreckt;
einen Aerosolerzeuger innerhalb des äußeren Rohrs (6), der in Gebrauch ein Kondensationsaerosol innerhalb des äußeren Rohrs (6) erzeugt;
eine Flüssigkeitszufuhr (22) innerhalb des äußeren Rohrs (6) zum Zuführen von flüssigem Material zum Aerosolerzeuger; und
ein Aerosolstrombildungselement innerhalb des äußeren Rohrs (6), das betreibbar ist, um einen Aerosolstrom zu erzeugen, wobei das Aerosolstrombildungselement ein Dichtungsring (10) ist, der so angeordnet ist, dass in Gebrauch das Aerosol durch eine zentrale Öffnung (63) des Dichtungsrings (10) gesaugt wird;
**dadurch gekennzeichnet, dass** der elektronische Rauchartikel (60) ferner umfasst:
einen Mundstückeinsatz in der Form eines mechanischen Aerosolwandlereinsatzes (104), der in Strömungsrichtung hinter dem Dichtungsring (10) angeordnet ist, wobei der mechanische Aerosolwandlereinsatz (104) dafür ausgelegt ist, auf den Aerosolstrom einen Aufprall auszuüben, während das Aerosol aus dem elektronischen Rauchartikel (60) gesaugt wird, wobei der mechanische Aerosolwandlereinsatz (104) drei längliche, im Allgemeinen nierenförmige, Austrittsöffnungen (112) aufweist, die sich rings um einen Umfang einer freiliegenden Endfläche (110) des mechanischen Aerosolwandlereinsatzes (104) erstrecken und sich rings um den Umfang einer Fläche des mechanischen Aerosolwandlereinsatzes (104) erstrecken, wobei die Fläche (102) stromaufwärts von der freiliegenden Endfläche (110) angeordnet ist, wobei die Fläche (102) von der zentralen Öffnung (63) beabstandet ist, wobei die Fläche (102) und die zentrale Öffnung (63) so angeordnet sind, dass in Gebrauch mindestens etwas von dem durch die zentrale Öffnung (63) gesaugten Aerosolstrom auf die Fläche (102) trifft und ein Merkmal des Aerosols verändert wird.

2. Elektronischer Rauchartikel nach Anspruch 1, wobei die drei länglichen Austrittsöffnungen (112), die sich rings um den Umfang der freiliegenden Endfläche (110) erstrecken, radial außerhalb der Fläche (102) angeordnet sind.

3. Elektronischer Rauchartikel nach Anspruch 2, wobei die Fläche (102) innerhalb eines zylindrischen Körpers (106) angeordnet ist, wobei die Fläche (102) von einem in Strömungsrichtung vorderen Gratteil (108) des zylindrischen Körpers (106) beabstandet ist, wobei der Gratteil (108) an dem Dichtungsring (110) anliegt, wobei ein Abstand zwischen der Fläche (102) und der zentralen Öffnung (63) beständig festgelegt ist.

4. Elektronischer Rauchartikel nach Anspruch 1, wobei das Merkmal des Aerosols mindestens eines von Aerosoltemperatur und Partikelgrößenverteilung ist.

5. Elektronischer Rauchartikel nach Anspruch 1, ferner umfassend:
ein inneres Rohr (62) innerhalb des äußeren Rohrs (6), wobei der Aerosolerzeuger ein im inneren Rohr (62) angeordnetes Heizelement (14) umfasst; und
einen Docht (28) in Kommunikation mit der Flüssigkeitszufuhr (22) und umgeben vom Heizelement (14), sodass in Gebrauch der Docht (28) dem Heizelement (14) flüssiges Material zuführt und das Heizelement (14) das flüssige Material auf eine Temperatur erhitzt, die ausreicht, um das flüssige Material zu verdampfen und das Aerosol im inneren Rohr (62) zu bilden, wobei die Flüssigkeitszufuhr (22) in einem äußeren Ringraum (620) zwischen dem äußeren Rohr (6) und dem inneren Rohr (62) enthalten ist.

6. Elektronischer Rauchartikel nach Anspruch 1, wobei die Fläche (102) porös ist und Partikel einer vorbestimmten Größe auf der Fläche (102) festgehalten werden können.

7. Elektronischer Rauchartikel nach Anspruch 1, wobei die Fläche (102) konkav ist.

8. Elektronischer Rauchartikel nach Anspruch 5, wobei der elektronische Rauchartikel (60) einen ersten Abschnitt (70) umfasst, der an einem zweiten Abschnitt (72) anbringbar ist, und wobei der Docht (28), das Heizelement (14) und die Flüssigkeitszufuhr (22) im ersten Abschnitt (70) enthalten sind und eine Stromversorgung (1) im zweiten Abschnitt (72) enthalten ist.

9. Elektronischer Rauchartikel nach Anspruch 1, wobei die Flüssigkeitszufuhr (22) einen Nikotingehalt von mehr als 2 Gewichtsprozent bezogen auf das Gewicht der Flüssigkeitszufuhr (22) hat.

10. Elektronischer Rauchartikel nach Anspruch 1, wobei jede der drei länglichen Austrittsöffnungen (112) eine Länge von etwa 2 mm bis etwa 5 mm und eine Breite von etwa 0,5 mm bis etwa 1 mm aufweist.

11. Elektronischer Rauchartikel nach Anspruch 1, wobei der mechanische Aerosolwandlereinsatz (104) aus einer Kunststoff- oder Metallfolie gebildet ist.

## Revendications

1. Article à fumer électronique (60) comprenant :
un tube externe (6) qui s'étend dans une direction longitudinale ;
un générateur d'aérosol à l'intérieur du tube externe (6) qui, en utilisation, produit un aérosol de condensation à l'intérieur du tube externe (6) ;
une alimentation en liquide (22) à l'intérieur du tube externe (6) pour délivrer une matière liquide au générateur d'aérosol ; et
un élément de formation de flux d'aérosol à l'intérieur du tube externe (6) qui est fonctionnel pour produire un flux d'aérosol, dans lequel l'élément de formation de flux d'aérosol est un joint d'étanchéité (10) qui est agencé de telle sorte que, en utilisation, l'aérosol soit aspiré au travers d'un orifice central (63) du joint d'étanchéité (10) ;
**caractérisé en ce que** l'article à fumer électronique (60) comprend en outre :
un insert d'extrémité d'embouchure sous la forme d'un insert de convertisseur d'aérosol mécanique (104) qui est disposé en aval dudit joint d'étanchéité (10), ledit insert de convertisseur d'aérosol mécanique (104) étant agencé pour imprimer un impact sur ledit flux d'aérosol lorsque ledit aérosol est en train d'être aspiré dudit article à fumer électronique (60), l'insert de convertisseur d'aérosol mécanique (104) incluant trois orifices de sortie allongés généralement réniformes (112) qui s'étendent de façon circonférentielle sur un périmètre d'une surface d'extrémité exposée (110) de l'insert de convertisseur d'aérosol mécanique (104) et s'étendant de façon circonférentielle sur un périmètre d'une face de l'insert de convertisseur d'aérosol mécanique (104), ladite face (102) étant disposée en amont de ladite surface d'extrémité exposée (110), ladite face (102) étant espacée dudit orifice central (63), ladite face (102) et ledit orifice central (63) étant agencés de telle sorte que, en utilisation, au moins une certaine partie dudit flux d'aérosol qui est aspiré au travers dudit orifice central (63) frappe ladite face (102) et qu'une caractéristique dudit aérosol soit modifiée.

2. Article à fumer électronique selon la revendication 1, dans lequel les trois orifices de sortie allongés (112) qui s'étendent de façon circonférentielle sur le périmètre de la surface d'extrémité exposée (110) sont disposés radialement vers l'extérieur de ladite face (102).

3. Article à fumer électronique selon la revendication 2, dans lequel ladite face (102) est disposée à l'intérieur d'un corps cylindrique (106), ladite face (102) est espacée d'une partie de bord amont (108) dudit corps cylindrique (106), ladite partie de bord (108) venant en butée contre ledit joint d'étanchéité (110), dans lequel un espacement entre ladite face (102) et ledit orifice central (63) est établi de façon constante.

4. Article à fumer électronique selon la revendication 1, dans lequel la caractéristique dudit aérosol est au moins l'une parmi une température d'aérosol et une distribution de tailles de particules.

5. Article à fumer électronique selon la revendication 1, comprenant en outre :
un tube interne (62) à l'intérieur du tube externe (6), dans lequel le générateur d'aérosol comprend un moyen de chauffage (14) qui est situé dans le tube interne (62) ; et
une mèche (28) en communication avec l'alimentation en liquide (22) et entourée par le moyen de chauffage (14) de telle sorte que, en utilisation, la mèche (28) délivre de la matière liquide au moyen de chauffage (14) et que le moyen de chauffage (14) chauffe la matière liquide jusqu'à une température suffisante pour vaporiser la matière liquide et pour former un aérosol dans le tube interne (62), dans lequel l'alimentation en liquide (22) est contenue dans un espace annulaire externe (620) entre le tube externe (6) et le tube interne (62).

6. Article à fumer électronique selon la revendication 1, dans lequel la face (102) est poreuse et des particules d'une taille prédéterminée peuvent être retenues sur la face (102).

7. Article à fumer électronique selon la revendication 1, dans lequel la face (102) est concave.

8. Article à fumer électronique selon la revendication 5, dans lequel l'article à fumer électronique (60) comprend une première section (70) qui peut être attachée à une seconde section (72) et dans lequel la mèche (28), le moyen de chauffage (14) et l'alimentation en liquide (22) sont contenus dans la première section (70) et une alimentation électrique (1) est contenue dans la seconde section (72).

9. Article à fumer électronique selon la revendication 1, dans lequel l'alimentation en liquide (22) présente une teneur en nicotine supérieure à 2 % en poids sur la base du poids de l'alimentation en liquide (22).

10. Article à fumer électronique selon la revendication 1, dans lequel chacun des trois orifices de sortie allongés (112) présente une longueur allant d'environ 2 mm à environ 5 mm et une largeur allant d'environ 0,5 mm à environ 1 mm.

11. Article à fumer électronique selon la revendication 1, dans lequel l'insert de convertisseur d'aérosol mécanique (104) est formé par une feuille en matière plastique ou en métal.
